(19) Europäisches Patentamt
European Patent Office
Office européen des brevets

(11) Veröffentlichungsnummer: **0 193 887**
**B1**

(12) # EUROPÄISCHE PATENTSCHRIFT

(45) Veröffentlichungstag der Patentschrift: **26.07.89**

(51) Int. Cl.⁴: **C07H 13/12,** C07H 15/04, A61K 31/70

(21) Anmeldenummer: **86102609.4**

(22) Anmeldetag: **28.02.86**

(54) **Substituierte O-Sulfonyl-Glycosylamide, Verfahren zu ihrer Herstellung sowie ihre Verwendung als Arzneimittel.**

(30) Priorität: **07.03.85 DE 3508025**

(43) Veröffentlichungstag der Anmeldung: **10.09.86 Patentblatt 86/37**

(45) Bekanntmachung des Hinweises auf die Patenterteilung: **26.07.89 Patentblatt 89/30**

(84) Benannte Vertragsstaaten: **AT BE CH DE FR GB IT LI NL SE**

(56) Entgegenhaltungen:
**EP-A- 0 091 645**
**EP-A- 0 143 385**
**US-A- 2 808 404**

**CARBOHYDRATE RESEARCH, Band 135, Nr. 2, 15. Januar 1985, Seiten 219-229, Elsevier Science Publishers B.V., Amsterdam, NL; Z. SMIATACZ et al.: "Nucleophilic displacement reactions of some N-acetyl-N-aryl-beta-D-xylopyranosylamines"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Krüger, Bernd-Wieland, Dr., Sillerstrasse 49, D-5600 Wuppertal 11(DE)**
Erfinder: **Hayauchi, Yutaka, Dr., Gustav-Freytag-Strasse 4, D-5090 Leverkusen 1(DE)**
Erfinder: **Lockhoff, Oswald, Dr., Morgengraben 14, D-5000 Koeln 80(DE)**
Erfinder: **Stadler, Peter, Dr., Am Ideck 8, D-5657 Haan 1(DE)**
Erfinder: **Metzger, Karl Georg, Dr., Pahlkestrasse 75, D-5600 Wuppertal 1(DE)**
Erfinder: **Stünkel, Klaus Georg, Dr., Am Eckbusch 55, D-5600 Wuppertal 1(DE)**
Erfinder: **Zeiler, Hans-Joachim, Dr., Elsbeeker Strasse 46, D-5620 Velbert 15(DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Verbindungen der allgemeinen Formel I

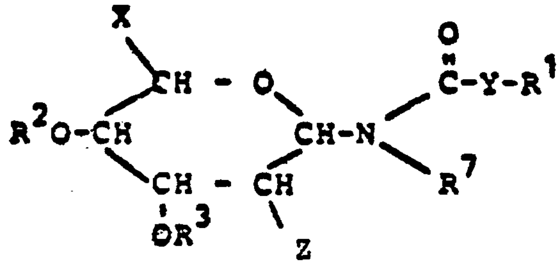

(I)

in welcher
X für Wasserstoff oder den Rest $-CH_2OR^5$ steht,
Z für $OR^4$ oder NHV steht, wobei,
V Wasserstoff oder 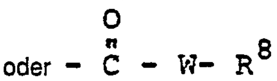 bedeutet,
Y und W gleich oder verschieden sind und für Sauerstoff, Schwefel, N-H oder $CH_2$ stehen,
$R^2$, $R^3$, $R^4$ und $R^5$ gleich oder verschieden sind und für Wasserstoff oder den Rest

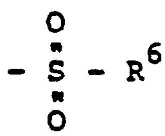

stehen, mit der Maßgabe, daß mindestens einer der Reste $R^2$, $R^3$, $R^4$ und $R^5$ die Bedeutung

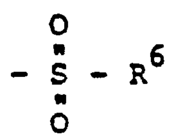

hat und
$R^1$, $R^6$, $R^7$ und $R^8$ gleich oder verschieden sind und für einen gegebenenfalls sustituierten Kohlenwasserstoffrest mit bis zu 50 Kohlenstoffatomen stehen,
Verfahren zu ihrer Herstellung und ihre Verwendung als Arzneimittel. Strukturell analoge Verbindungen aber ohne die O-Sulfonyl-Gruppe werden in der EP 91 645 beschrieben.

Unter Kohlenwasserstoffrest in der Bedeutung der Reste $R^1$, $R^6$, $R^7$, $R^8$ wird erfindungsgemäß ein geradkettiger oder verzweigter Alkylrest, ein geradkettiger oder verzweigter, ein- oder mehrfach ungesättigter Alkenylrest, ein gesättigter oder ungesättigter alicyclischer Rest oder ein aromatischer Rest (Aryl) verstanden. Diese Bedeutungen können innerhalb desselben Restes $R^1$, $R^6$, $R^7$ und $R^8$ auch gemeinsam auftreten, d.h. beispielsweise als Alkylcycloalkyl, Arylalkyl, Alkylaryl, Alkenylcycloalkyl, etc.

In den Kohlenwasserstoffresten $R^1$, $R^6$, $R^7$, $R^8$ können auch einzelne, im allgemeinen bis zu 5, vorzugsweise 1, 2 oder 3 Methylen- oder Methingruppen durch 0, S und/oder N ersetzt sein. Bei einer Unterbrechung der Kette durch N trägt dieser Stickstoff entweder H oder einen $C_1$-$C_{20}$-Alkylrest oder einen -CO-Alkylrest, wobei diese Alkylgruppe 1-20 C-Atome aufweist.

Bevorzugt steht $R^6$ für einen Alkylrest mit 1 bis 21 C-Atomen, vorzugsweise mit 1-4 C-Atomen oder für einen gegebenenfalls substituierten Arylrest mit bis zu 21 C-Atomen, vorzugsweise mit bis zu 7 C-Atomen.

Beispielhalft für gesättigte Reste seien hier benannt Methyl, Ethyl, Propyl, i-Propyl, Butyl, i-Butyl. Beispiele für Aryl sind Halogen-, vorzugsweise Chlor- oder Brom-substituiertes, Nitro-, Cyano-, Amido-$C_1$-$C_4$-Alkyl- oder $C_1$-$C_4$-Alkoxy-substituiertes Phenyl, Naphthyl oder Biphenyl.

Bevorzugt stehen $R^1$, $R^7$ und $R^8$ wahlweise für Alkyl- oder Alkenylreste mit 1 bis 21 Kohlenstoffatomen, vorzugsweise mit 9 bis 21 C-Atomen. Beispielhaft für gesättigte Reste seien hier genannt Methyl, Ethyl, Propyl, i-Propyl, Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Ethylpentyl, Methyldecyl, i-Propyldecyl, Methyltridecyl, Eicosyl, Tetracosyl, Triacontyl, Pentahexadecyl, 1-Dodecylhexadecyl, 2-Dodecylhexadecyl, 3-Dodecylhexadecyl, 1-Hexadecyl octadecyl,2-Hexadecyloctadecyl, 3-Hexadecyloctadecyl, 4-Hexadecyloctadecyl, 1-Octadecyleicosyl und 2-Octadecyleicosyl.

Ungesättigte Reste sind beispielsweise Vinyl, Propenyl-1, Propenyl-2, i-Butenyl, Butenyl-1, Butenyl-2, 1-Pentenyl, 2-Pentenyl, 3- Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen, ungesättigten Rest bevorzugt, speziell die ein- oder zweifach ungesättigten Alkenyle mit 9-21 C-Atomen.

Die ungesättigten Kohlenwasserstoffreste können dabei als reine cis- oder trans-Isomere oder auch als Isomerengemische vorliegen.

Beispielhaft für Cycloalkyl seien genannt Cyclopentyl, Cyclohexyl, Decahydronaphthyl und Adamantyl.

Beispielhaft für Alkyl-cycloaklylreste seien genant Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Delcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexylhexyl, Cyclohexyldecyl, Cyclopentyl, Cyclohexylethyl, Cyclohexylcyclopentylethyl und Cyclohexylcyclohexylethyl.

Beispielhaft für Aryl seien genannt Phenyl, Tosyl, Naphtyl und Diphenyl.

Beispiel für Aralkyl für $R^1$, $R^7$ und $R^8$ sind Arylniedrigalkyl wie Benzyl, Phenethyl oder Phenylhexyl.

Die Reste $R^1$, $R^6$, $R^7$ und $R^8$ können substituiert sein, im allgemeinen 1-5-, vorzugsweise 1-3-fach. Als Substituenten kommen vorzugsweise die folgenden in Betracht: Halogen, vorzugsweise F, Cl oder Br, Armino, $C_1$-$C_6$-Aklylamino, Di-$C_1$-$C_6$-alkylamino, Oxo, OH, $C_1$-$C_6$-Alkoxy, SH, $C_1$-$C_6$-Alkylthio, $C_1$-$C_6$-Alkyl-COO und $C_1$-$C_6$-Alkyl-CO-NH.

Beispiele für Fälle, in denen die Kohlenwasserstoffreste $R^1$, $R^7$ und $R^8$ durch O, S und N bzw. entsprechende Atomgruppierungen unterbrochen oder z.B. durch diese Atome enthaltende Gruppen oder durch Halogenatome substituiert sind, sind Methoxyethyl, Ethoxyethyl, n-Propoxyethyl, n-Butoxyethyl, i-Propoxyethyl, i-Butoxyethyl, sek.-Butoxyethyl, Methoxyethoxyethyl, Ethoxyethoxyethyl, Propoxyethoxyethyl, i-Propoxyethoxyethyl, n-Butoxyethoxyethyl, i-Butoxyethoxyethyl, sek.-Butoxyethoxyethyl, Methoxyethoxyethoxyethyl, Ethoxyethoxyethoxyethyl, n-Propoxyethoxyethoxyethyl, i-Propoxyethoxyethoxyethyl, n-Butoxyethoxyethoxyethyl, i-Butoxyethyoxyethoxyethyl, sek.-Butyloxyethoxyethoxyethyl, wenn Y und/oder Z für Sauerstoff, Schwefel, N-H oder $CH_2$ stehen; Methoxyethoxy, Ethoxyethoxy, n-Propoxyethoxy, i-Propoxyethoxy, n-Butoxyethoxy, i-Butoxyethoxy, sek.-Butoxyethoxy, Methoxyethoxyethoxy, Ethoxyethoxyethoxy, n-Propoxyethoxyethoxy, i-Propoxyethoxyethoxy, n-Butoxyethoxyethoxy, i-Butoxyethoxyethoxy, sek.-Butoxyethoxyethoxy, wenn Y und/oder Z für $CH_2$ stehen; Hydroxyheptadecenyl, Oxobutyl, Amino-decyl-, N-Methylaminodecyl-, Fluormethyl-, ß-Hydroxytridecyl- oder Mercaptoethylrest.

Die Verbindungen der Formel I enthalten mehrere chirale C-Atome und liegen als optisch reine Diastereomere oder als Diastereomerengemische vor.

Die erfindungsgemäßen Verbindungen der Formel I sind also Carbonsäureamide bzw. N-alkylierte bzw. N-aralkylierte Carbonsäureamide, Carbamidsäure-, Thiocarbamidsäure- oder Harnstoffderivate, die an dem durch den obengenannten Rest $R^7$ substituierten Stickstoffatom zusätzlich N-glycosidisch, d.h. über das anomere Kohlenstoffatom gebunden einen einfachen Monosaccharidrest tragen, wobei eine oder meherer Hyrdoxygruppen im Saccharidrest mit einem Sulfonylrest versehen sind.

Besonders bevorzugt sind Verbindungen, in denen nur einer der Rest $R^2$, $R^3$ $R^4$ oder $R^5$ die Bedeutung $SO_2$-$R^6$ hat, mit der Maßgabe, daß die anderen Reste $R^2$, $R^3$, $R^4$ und/oder $R^5$ für Wasserstoff stehen und $R^6$ und Z die oben genannte Bedeutung haben.

Ganz besonders bevorzugt sind Verbindungen, in denen $R^2$, $R^3$ und $R^4$ für Wasserstoff stehen, und $R^5$ für den Rest $SO_2$-$R^6$ steht, wobei Z und $R^6$ die obengenannte Bedeutung haben.

Als Beispiele seien genannt:

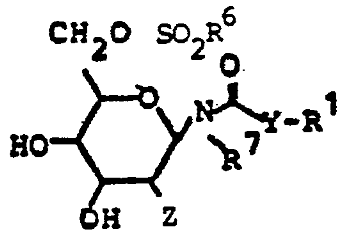

| Y | Z | $R^1$ | $R^6$ | $R^7$ |
|---|---|---|---|---|
| $CH_2$ | OH | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 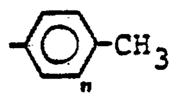 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 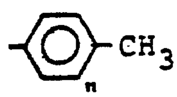 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | " | $(CH_2)_{11}CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 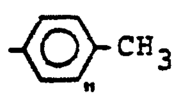 | $(CH_2)_{11}CH_3$ |
| " | " | " | " | $(CH_2)_{13}CH_3$ |

| Y | Z | $R^1$ | $R^6$ | $R^7$ |
|---|---|---|---|---|
| $CH_2$ | OH | $(CH_2)_{11}CH_3$ | 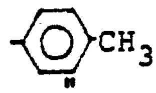 | $(CH_2)_{15}CH_3$ |
| " | " | " | " | $(CH_2)_{17}CH_3$ |
| O | " | " | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 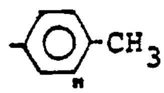 | $(CH_2)_{11}CH_3$ |
| " | " | " | " | $(CH_2)_{15}CH_3$ |
| " | " | " | " | $(CH_2)_{15}CH_3$ |
| " | " | " | " | $(CH_2)_{17}CH_3$ |
| $CH_2$ | " | $(CH_2)_{13}CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 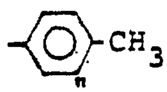 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | " | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 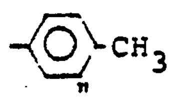 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| $CH_2$ | OH | $(CH_2)_{15}CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | ". | 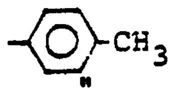 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | " | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | 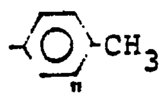 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $NH_2$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| $CH_2$ | $NH_2$ | $(CH_2)_{13}CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_9CH_3$ | 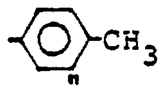 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| $CH_2$ | $NHCOCH_3$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{15}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |
| $CH_2$ | " | $(CH_2)_9CH_3$ | 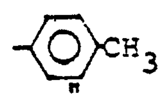 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| $CH_2$ | $NHCOCH_3$ | $(CH_2)_{13}CH_3$ | 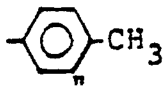 | $(CH_2)_{11}(CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |
| " | " | $(CH_2)_9CH_3$ | 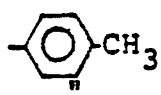 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| O | $NHCOCH_3$ | $(CH_2)_{11}CH_3$ | 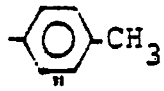 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}(CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | $NH_2$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |

| Y | Z | $R^1$ | $R^6$ | $R^7$ |
|---|---|---|---|---|
| O | $NH_2$ | $(CH_2)_9CH_3$ | 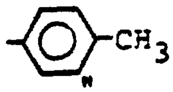 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| $CH_2$ | $NHCO(CH_2)_{10}CH_3$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |
| " | " | $(CH_2)_9CH_3$ | 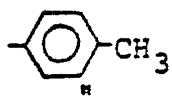 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}(CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R'$ |
|---|---|---|---|---|
| $CH_2$ | $NHCO(CH_2)_{10}CH_3$ | $(CH_2)_{15}CH_3$ | 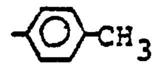 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |
| " | " | $(CH_2)_9CH_3$ | 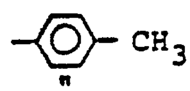 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

| Y | Z | $R^1$ | $R^6$ | $R^7$ |
|---|---|---|---|---|
| O | $NHCO(CH_2)_{10}CH_3$ | $(CH_2)_{13}CH_3$ | 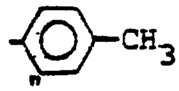 | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $NHCONHCH_3$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17})CH_3$ |
| $CH_2$ | " | $(CH_2)_9CH_3$ | 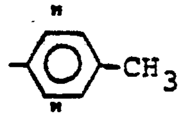 | $(CH_2)_{11}CH_3$ |
| " | " | " | " | $(CH_2)_{13}CH_3$ |
| " | " | " | " | $(CH_2)_{15}CH_3$ |

| Y | Z | $R^1$ | $R^6$ | $R^7$ |
|---|---|---|---|---|
| $CH_2$ | $NHCONHCH_3$ | $(CH_2)_9CH_3$ | 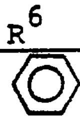-$CH_3$ | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}(CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | $NHCONHCH_3$ | $(CH_2)_9CH_3$ | $CH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |

| Y | Z | $R^1$ | $R^6$ | $R^7$ |
|---|---|---|---|---|
| O | $NHCONHCH_3$ | $(CH_2)_{15}CH_3$ | 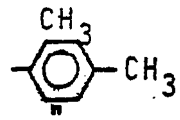 | $(CH_2)_{17})CH_3$ |
| " | " | $(CH_2)_9CH_3$ | | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{22}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}(CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

Die Erfindung betrifft auch Verfahren zur Herstellung der Verbindungen der Formel I. Hierbei setzt man Verbindungen der Formel II

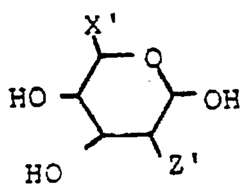

(II)

in der
X' für Wasserstoff oder $-CH_2OH$ steht, und
Z' für OH oder $NHCO-W-R^8$ steht, wobei $R^8$ und W die obengenannte Bedeutung haben,
entweder in freier, d.h. ungeschützter Form, oder in Form geschützter, gegebenenfalls aktivierter Derivate zunächst mit einer Aminoverbindung $R^7-NH_2$, entweder in freier Form oder in Form eines geigneten Säureadditionssalzes mit der vorstehend beschriebenen Bedeutung für $R^7$, um und acyliert anschließend das dabei erhaltene Glykosylamin mit einem - wie bei Acylierungsreaktionenüblich - aktivierten, an funktionellen Gruppen gegebenenfalls geschützten Carbonsäure-, Kohlensäure, Thiokohlensäurederivat oder Isocyanat, spaltet in dem so erhaltenen Reaktionsprodukt gegebenenfalls vorhandene Schutzgruppen selektiv oder vollständig ab und setzt in einem zweiten Verfahrenschritt das so erhaltene, an mindestens einer Hydroxygruppe freie, d.h. ungeschützte, Zwischenproduckt mit einem aktivierten, an funktionellen Gruppen gegebenenfalls geschützten Sulfonsäurederivat um. Nach Abspaltung gegebenenfalls vorhandener Schutzgruppen erhält man auf diese Weise die erfindungsgemäßen Verbindungen der Formel I, die, falls erforderlich, durch Chromatographie, Umkristallisieren, Extraktion, o.ä. gereinigt werden können.

In einer bevorzugten Ausführungsform des erfindungsgemäßen Verfahrens wird in an sich bekannter Weise in einem ersten Verfahrensschritt der unblockierte Zucker der Formel II in einem geeigneten Lösungsmittel, oder auch ohne Lösungsmittel, gegebenenfalls in Gegen wart eines Katalysators bei Temperaturen zwischen 0°C und 80°C mit 1 bis 10 Äquivalenten des betreffenden Amins $R^7-HN_2$ umgesetzt

und man erhält gewöhnlich in hohen Ausbeuten nach Aufarbeitung die betreffenden Glykosylamine als amorphe oder kristalline Feststoffe oder als zähe Sirupe.

Im zweiten Verfahrensschritt wird dann das Glykosylamin mit 1 bis 10 Äquivalenten eines Acylderivates der Formel $R_1$-Y-CO-X", in der $R_1$ und Y die obengenannte Bedeutung besitzen und X" Halogen oder eine bei Acylierungsreaktionen gebräuchliche Abgangsgruppe, vorzugsweise einen aktivierenden Esterrest, oder eine Gruppe O-OC-Y-$R_1$ mit der obigen Bedeutung für Y und $R_1$ bezeichnet, bzw. 1 bis 10 Äquivalenten eines Isocyanates der Formel $R^1$-NCO umsetzt, wobei man in einem organischen oder wäßrig-organischen Lösungsmittel bei Temperaturen zwischen -30°C und 80°C, gegebenenfalls in Gegenwart einer Base, arbeitet und nach beendeter Umsetzung das Reaktionsprodukt in üblicher Weise aufarbeitet.

In einem dritten Verfahrensschritt wird dann das so erhaltene, am Stickstoff umgesetzte Derivat, in geschützter oder ungeschützter Form mit 1 bis 10 Äquivalenten eines Sulfonylderivates der Formel

$$R^6 - \overset{\overset{\displaystyle O}{\|}}{\underset{\underset{\displaystyle O}{\|}}{S}} - X''' \quad ,$$

in der $R^6$ die obengenannte Bedeutung besitzt und X''' für Halogen steht, umgesetzt, wobei man in einem organischen oder wäßrigorganischen Lösungsmittel bei Temperaturen zwischen -30°C und 80°C, gegebenenfalls in Gegenwart einer Base, arbeitet und nach beendeter Umsetzung das Reaktionsprodukt in üblicher Weise aufarbeitet.

Der erste Verfahrensschritt bei der Herstellung der erfindungsgemäßen Verbindungen der Formel I ist somit die Umsetzung eines Zuckers mit einem Amin des Types $R^7$-$NH_2$ am anomeren Kohlenstoffatom unter Wasserabspaltung zu dem betreffenden Glykosylamin.

Amine $R^7$-$NH_2$, die bei Raumtemperatur flüssig sind, können mit dem Zucker direkt, d.h. ohne Lösungsmittel umgesetzt werden. Hierbei arbeitet man bei Temperaturen zwischen 0°C und 100°C vorzugsweise bei 25°C bis 70°C,. Als Katalysatoren sind Mineralsäuren wie z.B. Salzsäure, Schwefelsäure oder Salpetersäure oder kurzkettige Carbonsäuren wie Essigsäure oder Propionsäure geeignet, die man in Mengen von 0,001 bis 0,05 Äquivalenten einsetzt.

In jedem Fall ist es möglich, und bei Aminen $R^7$-$NH_2$, die bei Raumtemperatur fest sind, auch zu bevorzugen, die Herstellung der Glykosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran und Dioxan, sowie auch Dimethylformamid, wobei - außer bei der Verwendung der Alkohole - der Zusatz von Wasser zu bevorzugen ist. Darüber hinaus ist vorzugsweise bei kurzkettigen Aminen $R^7$-$MH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen -10°C und 120°C vorzugsweise zwischen 30°C und 70°C.

Das betreffende Verdünnungsmittel kann wahlweise vor oder während der Reaktion zugegeben werden. Bei langkettigen Aminen $R^7$-$NH_2$, ist eine Zugabe vor der Reaktion zu bevorzugen.

Die wie vorstehend beschrieben hergestellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden, gegebenenfalls vorhandenes überschüssiges Amin $R^7$-$MH_2$ kann durch Waschen oder Umkristallisieren des Produktes auf an sich bekannte Weise entfernt werden.

Der zweite Verfahrensschritt bei der Herstellung der erfindungsgemäßen Verbindungen der Formel I ist die selektive N-Acylierung eines wie vorstehend beschrieben erhaltenen Glykosylamins mit einem Acylderivat der Formel $R_1$-Y-CO - X", mit der vorstehend angegebenen Bedeutung von $R_1$, Y und X" oder einem Isocyanat der Formel $R^1$-NCO.

Der dritte Verfahrensschritt bei der Herstellung der erfindungsgemäßen Verbindungen der Formel I ist die selektive O-Sulfonylierung eines vorstehend beschriebenen geschützten oder ungeschützen N-acylierten Aminoglykosids mit einem Sulfonylderivat der Formel

$$R^6 - \overset{\overset{O}{\|}}{\underset{\underset{O}{\|}}{S}} - X''' ,$$

mit der vorstehend angegebenen Bedeutung von $R^6$ und X'''.

Als an sich bekannte Carboxylderivate $R_1$-Y-CO-X" sind zu bevorzugen Anhydride, aktivierte Ester und Säurehalogenide, vorzugsweise Chloride, als an sich bekannte Sulfonylderivate $R^6$-$SO_2$-X''' Säurehalogenide, vorzugsweise Chloride.

Diese Verbindungen werden vorzugsweise in Gegenwart eines Verdünnungsmittels mit den Glykosylaminen bzw. -amiden umgesetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern. Man kann sowohl in organischen Lösungsmitteln wie Ethern, z.B. Tetrahydrofuran und Dioxan, oder Alkoholen z.B. Ethanol und Propanol, oder Ketonen, z.B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Verbindungen $R^1$-Y-COX"/$R^6$ $SO_2$-X''' bzw. $R^1$-NCO werden in 1 bis 10 Äquivalenten, bezogen auf Glykosylamin/ Glykosylamid eingesetzt.

Die Reaktionen können vorzugsweise bei Verwendung von Säurehalogeniden und -anhydriden, in Gegenwart basischer Hilfsstoffe durchgeführt werden. Verwendet werden können alle in der organischen Synthese üblichen basischen Verbindungen wie z.B. tertiäre aliphatische oder auch aromatische Amine oder aber Alkali- und Erdalkalihydroxide bzw. -carbonate wie Natronlauge, Natriumcarbonat oder Calciumcarbonat.

Sie werden bei Temperaturen zwischen etwa -30°C und +80°C, vorzugsweise zwischen -10°C und +20°C durchgeführt.

In den Fällen, in denen eine selektive Umsetzung einer Hydroxylgruppe nicht durchgeführt werden kann, weil entweder eine weniger reaktive sekundäre Hydroxylgruppe des Zuckerrestes bei Vorhandensein einer reaktiveren primären Hydroxylgruppe mit dem Rest

$$- \overset{\overset{O}{\|}}{\underset{\underset{O}{\|}}{S}} - R^6$$

mit der obengenannten Bedeutung für $R^6$ umgesetzt werden soll, oder aber eine sekundäre Hydroxylgruppe selektiv bei Vorhandensein anderer sekundärer Hydroxylgruppen mit dem Rest

$$- \overset{\overset{O}{\|}}{\underset{\underset{O}{\|}}{S}} - R^6$$

umgesetzt werden soll, oder aber eine weniger reaktive Hydroxylgruppe bei Vorhandensein einer reaktiven Aminogruppe mit dem Rest

$$- \overset{\overset{O}{\|}}{\underset{\underset{O}{\|}}{S}} - R^6$$

mit der obengenannten Bedeutung für $R^6$ umgesetzt werden soll, ist der eigentlichen Sulfonylierungsreaktion eine Reihe von Schutzgruppenoperationen vorzuschalten, bei der die selektiv mit dem Rest

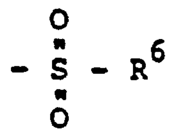

umzusetzende Hydroxylgruppe am Ende der Blockierungsreaktionen frei, d.h. unsubstituiert, vorliegt. Die nicht umzusetzenden Hydroxylgruppen bzw. Aminogruppe müssen also vor der Sulfonylierung blockiert werden.

Geeignete Schutzgruppen für Zuckerderivate sind in der einschlägigen Literatur beschrieben (z.B. C.B. Reese, in Protecting Groups in Org. Chem., 1973, p 95 - p 143; Plenum Press). Es können alle in der Zuckerchemie verwendeten Schutzgruppen und Kombinationen daraus benutzt werden.

Geeignete Schutzgruppen sind z.B. Ester wie Acetyl, Benzoyl, Pivaloyl, p-Methoxybenzoyl, Ether wie Benzyl, p-Methoxybenzyl, Allyl, 1-Propenyl, Alkylidenverbindungen wie Ethyliden, Isopropyliden, Benzyliden, Orthoester wie 1-Methoxy-ethyliden, 1-Ethoxy-ethyliden, Silylether wie Trimethylsilyl, t-Butyldimethylsilyl, Organometallverbindungen wie Borsäureester oder Zinnether oder Zinnketale wie Tributylstannyl oder Dibutylstannyliden.

Die durch diese Schutzgruppen blockierten Kohlenhydratderivate werden dann an der/den noch freien Hydroxylgruppe(n) in einem geeigneten Lösungsmittel mit der Gruppe

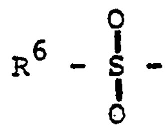

umgesetzt. Geeignete Lösungsmittel und geeignete Verfahren zur Sulfonylierung sind obengenannt.

Die auf diese Weise erhaltenen O-sulfonylierten Amide, Harnstoffe, Carbamate bzw. Thiocarbamate werden nach an sich bekannten Verfahren in Form von kristallinen oder amorphen Feststoffen oder als zähe Sirupe isoliert und, wenn notwendig, durch Umkristallisation, Chromatographie, Extraktion usw. gereinigt.

Im Falle von Verbindungen mit geschützten Hydroxyl- bzw. Aminogruppen im Glykosylteil können die Schutzgruppen in an sich bekannter Weise abgespalten werden.

Das folgende Formelschema soll eine der bevorzugten Ausführungsformen der erfindungsgemäßen Darstellung von Verbindungen der Formel I beispielhaft erläutern:

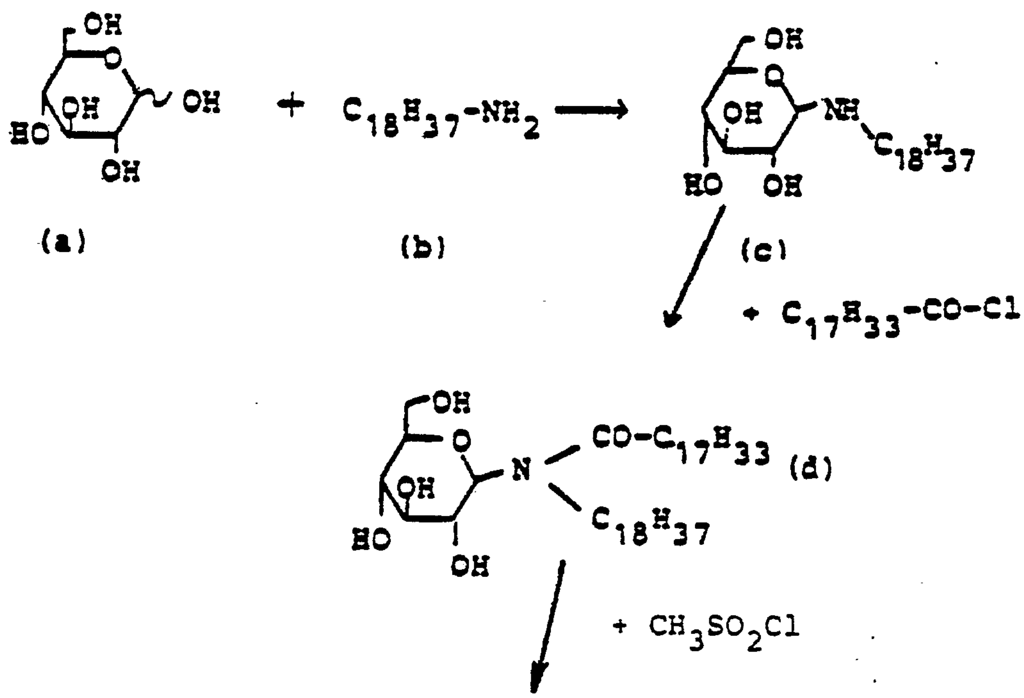

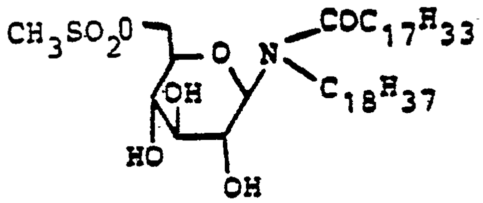

(I)

Im ersten Verfahrensschritt wird Glucose (a) mit Octadecylamin (b) zu N-Octadecyl-ß-D-glycopyranosylamin (c) umgesetzt, das im zweiten Verfahrensschritt mit Ölsäurechlorid zu N-Octadecyl-N-oleoyl-ß-D-glucopyranosylamin (d) acyliert wird. Im dritten Verfahrensschritt wird dann mit Methansulfonsäurechlorid in Position 6 O-sulfonyliert und man erhält N-Octadecyl-N-oleoyl-(6-methylsulfonyl-ß-D-glucopyranosyl)-amin (I).

Zum Gegenstand der Erfindung gehören auch Salze der Verbindungen der Formel I. Dabei handelt es sich in erster Linie um üblicherweise pharmazeutisch verwendbare, nicht-toxische Salze, z.B. Alkalimetall- oder Ammoniumsalze, oder Hydrochloride, Hydroacetate oder Chloride oder Acetate.

Die Verbindungen der Erfindung weisen eine ausgeprägte Abwehr-steigernde Wirkung auf. Es wurde gefunden, daß die Verbindungsklasse die Antikörpersynthese des Immunsystems Antigen-spezifiisch steigert und darüber hinaus die unspezifische wirtseigene Abwehr verstärkt. Diese Ergebnisse wurden anhand der nachfolgenden Versuchsanordnung erhalten.

Steigerung der primären humoralen Immunität in vitro gegen Schaferythrozyten (SE).

Es ist experimentell möglich, die Entwicklung einer humoralen Immunantwort mit heterologen roten Blutzellen durch primäre Immunisation von Maus-Milzzellen in Suspensionskulturen in vitro einzuleiten (R. I. Mishell und R. W. Dutton, J. Exp. Med. 126, 423 (1967)). Hierzu werden Balb/c Maus-Milzzellen für 5 Tage in Gegenwart von Antigen (SE) und der Testsubstanz kultiviert. Die Zellen werden geerntet, gewaschen und zusammen mit dem Antigen und Komplement in semisolidem Agar ausplattiert und für 2 Stunden bei 37°C inkubiert (N. K. Jeren, A. A. Nordin und C. Henry, "Cell bound Antibodies", eds. Amos and Koprowski, Wistar Inst. Press, Philadelphia, USA, pp 109 (1963)). Die Antigen-Sensibilisierung von Maus-Lymphozyten in der Primärkultur resultiert in der Synthese und Freisetzung von Antikörper. Die spezifischen, sezernierten Antikörper binden sich an das SE-Antigen und lysieren diese Zellen durch die Gegenwart des Komplements (Plaque-Bildung). Substanzen der vorliegenden Verbindungsklasse sind in der Lage, dosisabhängig im Bereich 3-100 µg/ml die Zahl der Antikörper-bildenden Zellen zu steigern (Tabelle 1).

Tabelle 1: Wirkung ausgewählter O-Sulfonyl-Glycosylamid-Analoga der vorliegenden Verbindungsklassen auf die Antikörpersynthese in vitro.

Tabelle 1: Wirkung ausgewählter O-Sulfonyl-Glycosylamid-Analoga der vorliegenden Verbindungsklassen auf die Antikörpersynthese in vitro.

| Substanz | Antikörper sezernierende Zellen/Kultur in Abhängigkeit von der Dosis (µg/ml) | | | | | |
|---|---|---|---|---|---|---|
| Beispiel Nr. | 0 | 1 | 3 | 10 | 30 | 100 |
| 11 | 1460 | 1840 | 4380 | 4040 | 12 320 | n. d.[1] |
| 8 | 1135 | 3060 | n. d. | 2890 | n. d. | 3240 |
| 10 | 2560 | 2500 | 3020 | 3840 | 6400 | 9560 |
| 14 | 495 | 5380 | 6140 | 11 500 | 11 520 | 13 880 |

[1] nicht durchgeführt

Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s.c.) immunisiert. Bei suboptimaler Antigenstimulation wurde nur eine geringe Zahl von Lymphozyten der Tiere zur Antikörpersynthese angeregt. Die zusätzliche Behandlung der Tiere mit Verbindungen der genannten Beispiele der vorliegenden Erfindung ist in der Lage, bei einer einmaligen Applikation von 10-30 mg/kg

subcutan den Antikörpertiter im Serum der Tiere signifikant zu steigern. Die Antikörpertiterbestimmung erfolgt durch indirekte Hämagglutination am Tag 10. Der Effekt der Behandlung wird durch den geometrischen Mittelwert der $log_2$-Titer ausgedrückt.

Tabelle 2: Adjuvantive Wirkung ausgewählter, erfindungsgemäßer Verbindungen in vivo am Beispiel des löslichen Antigens Ovalbumin.

**Tabelle 2: Adjuvantive Wirkung ausgewählter, erfindungsgemäßer Verbindungen in vivo am Beispiel des löslichen Antigens Ovalbumin.**

| Substanz | Dosis (mg/kg) | | | |
|---|---|---|---|---|
| Beispiel Nr. | 0 | 3 | 10 | 30 |
| | Hämaggl. Titer (log 2) | | | |
| 7 | 4.0 | 5.4[1)] | 5.8 | 6.4 |
| 8 | 4.2 | 5.4 | 5.6 | 6.2 |
| 15 | 4.4 | 5.0[1)] | 6.0 | 6.8 |
| 14 | 4.0 | 4.6[2)] | 6.2 | 6.6 |

1) alle Werte signifikant gesteigert ($p \leq 0.01$)
2) nicht signifikant

Der immunstimulierende Effekt der genannten Verbindungen ist im Gegenwatz zu anderen, z.B. bakteriellen Immunstimulantien wie LPS aus Gram-negativen Bakterien Antigen-abhängig, d.h. die Substanzen bewirken überraschenderweise nur in Verbindung mit einem antigenen Reiz (hier SE oder Ovalbumin) die Induktion der Antikörpersynthese. Sie haben im Gegensatz zu den erwähnten konventionellen Immunstimulantien keine mitogenen Eigenschaften.

Verträglichkeit

Obwohl Verbindungen der beschriebenen Art ihre potenzierende Wirkung an der Maus beispielsweise bereits nach einer Einzeldosis von 10 mg/kg i.p., oder peroral entfalten, werden auch bei Applikation von 100 mg/kg keine toxischen Effekte beobachtet. Die genannten Stoffe verfügen deshalb über eine gute Verträglichkeit.

Die erfindungsgemäßen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen oder bei selektiven (antigenspezifischen) immunologischen Defekten, sowie bei angeborenen, aber auch erworbenen allgemeinen (d.h. nicht antigenspezifisch) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen erhöhen die Überlebensrate im Tiermodell der akuten bakteriellen Infektion.

Sie können allein als Prophylaktikum, zur Bekämpfung bestehender Infektionen oder in Kombination mit einer antibiotischen Therapie zur Steigerung der therapeutischen Wirkung von Antibiotika und Chemotherapeutika (z.B. Penicilline, Cephalosporine, Aminoglykoside etc.) bei infizierten Menschen und Tieren verwendet werden.

Versuchsbeschreibung

Es wurde gefunden, daß Infektionen der Maus mit pathogenen Keimen, die innerhalb von 24-48 Stunden zum Tod der Versuchstiere führen, durch eine prophylatkische Behandlung - bevorzugt intraperitoneal - mit 1-80 mg/kg der erfindungsgemäßen Verbindungen therapiert werden können. Dies trifft für eine ganze Reihe grampositiver (z.B. Staphylokokken) und gramnegativer (z.B. E.coli, Klebsiella, Proteus, Pseudomonas) Krankheitserreger zu.

Diese Aufzählung ist beispielhaft und keineswegs beschränkend aufzufassen. So überleben z.B. Mäuse, die mit dem pathogenen Stamm Klebsiella 63 infiziert worden waren, nach Behandlung (z.B. 18 Stunden vor Infektion) mit 10-40 mg/kg der erfindungsgemäßen Verbindung gemäß Beispiel 7, 8 und 11 zu 40 bis 100 % diese Infektion, während von den unbehandelten Kontrolltieren nur 0 bis 30 % überlebten.

In einem weiteren Versuchsmodell konnte gezeigt werden, daß die therapeutische Wirksamkeit von Antibiotika durch die erfindungsgemäßen Verbindungen gesteigert werden kann. So wurden Mäuse mit dem Stamm Pseudomonas W. infiziert. Diese Infektion führt bei den meisten Kontrolltieren innerhalb 24 Stunden zum Tode. Eine weitere Gruppe wurde mit 4 mg/kg Sisomicin 30 Stunden post infectionem behandelt. Es konnte gezeigt werden, daß in der Versuchsgruppe, die mit den erfindungsgemäßen Verbindungen (Beispiels siehe oben) 18 Stunden vor Infektion behandelt worden waren, die therapeutische Wirksamkeit des Sisomicins entscheidend verbessert werden konnte.

Die pharmazeutischen Präparate der vorliegenden Erfindung sind vorzugsweise Tabletten oder Gelatinekapseln, welche die Wirkstoffe zusammen mit Verdünnungsmitteln, z.B. Lactose, Dextrose, Saccharose, Mannit, Sorbit, Cellulose and/oder Schmiermitteln, z.B. Kieselerde, Talk, Stearinsäure oder Salze davon, wie Magnesium- oder Calciumstearat, und/oder Polyethylenglykol, enthalten. Tabletten enthalten eben falls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabiliser-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0, 1 % bis etwa 75% insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z.B. bakterielle, virale und parasitäre) und malignen Tumoren verwendet werden. Sie können ebenfalls als Adjuvantien bei der Vakzinierung, bei der Stimulierung von Phagocytose, bei der Dysregulierung des Abwehr- und Immunsystems verwendet werden.

Beispiele

Beispiel 1

N-Octadecyl-D-glucopyranosylamin

20 g Octadecylamin werden in 120 ml Ethanol gelöst und auf 70°C erwärmt. Es werden 11 g wasserfreie D-Glucose zugesetzt. Nachdem sich eine klare Lösung gebildet hat wird noch 15 min. bei 70°C weitergerührt. Es wird auf 10° abgekühlt und 15 min. stehengelassen. Der gebildete Kristallbrei wird abgesaugt, zweimal mit Ethanol gewaschen und im Vakuum getrocknet.

| Elementaranalyse: | | | |
|---|---|---|---|
| ber.: | C 66,8% | H 11,4% | N 3,2% |
| gef.: | C 67,4% | H 11,8% | N 3,7% |

Beispiel 2

N-Glucopyranosyl-N-octadecyl-dodecansäureamid

10 g der Verbindung aus Beispiel 1 werden in 20 ml Tetrahydrofuran aufgeschlämmt und nach Zusatz von 10 g Soda tropfenweise mit 10 g Dodecansäurechlorid in 10 ml Tetrahydrofuran versetzt. Nach beendeter Reaktion (dünnschichtchromatographische Kontrolle auf Kieselgel 60 in Toluol-Isopropanol 6:1) wird der Feststoff abfiltriert, das Filtrat im Vakuum zum Sirup eingedampft und das Rohprodukt säulenchromatographisch auf Kieselgel 60 mit dem Elutionsmittel Toluol-Isopropanol 10:1 gereinigt.
$\alpha_D$ = 8° (c - 1,0 in Dioxan)

Beispiel 3

N-Dodecyl-D-galactopyranosylamin

Herstellung gemäß Beispiel 1 aus D-Galactose und Dodecylamin.

| Elementaranalyse: | | | |
|---|---|---|---|
| ber.: | C 62,2% | H 10,7% | N 4,0% |
| gef.: | C 62,5% | H 10,2% | N 4,4% |

Beispiel 4

N-Galactopyranosyl-N-dodecyl-octadecansäureamid

Herstellung gemäß Beispiel 2 aus 10 g der Verbindung gemäß Beispiel 3 und 16 g Stearinsäurechlorid.
$\alpha_D$= 4,4° ( c = 1,0 in Dichlormethan)
Rf-Wert = 0,23 in Toluol/n-Propanol 4:1

Beispiel 5

N-Octadecyl-N-(D-glucopyranosyl)-decylurethan

9 g der Verbindung aus Beispiel 1 werden in 160 ml Tetrahydrofuran und 40 ml Ethanol aufgeschlämmt und mit 9 g Nitriumcarbonat versetzt. Zu dieser Suspension werden 5 g Chlorameisensäuredecylester, gelöst in 40 ml Tetrahydrofuran, während 20 min zugetropft. Nach beendeter Reaktion wird der Ansatz filtriert, der Filterrückstand mit Tetrahydrofuran nachgewaschen. Das Filtrat wird mit den Waschlösungen vereinigt und im Vakuum eingedampft. Der erhaltene Sirup wird chromatographische gereinigt (Laufmittel Dichlormethan/Methanol, 20:1)
Rf-Wert 0,37 in $CH_2Cl_2/CH_3OH$ 10:1

| Elementaranalyse: | | | |
|---|---|---|---|
| ber.: | C 68,3% | H 11,3% | N 2,3% |
| gef.: | C 68,4% | H 11,6% | N 2,4% |

Beispiel 6

N-Octadecyl-N-(D-glucopyranosyl)-N'-dodecylharnstoff

9 g der Verbindung aus Beispiel 1 werden in 160 ml Tetrahydrofuran und 40 ml Ethanol aufgeschlämmt. Zu dieser Suspension werden, 4,3 g Dodecylisocyanat, ge löst in 20 ml Tetrahydrofuran, während 20 min getropft. Nach beendeter Reaktion wird im Vakuum eingedampft und der erhaltene Sirup säulenchromatographisch gereinigt (Laufmittel Dichlormethan/Methanol, 15:1)
Rf-Wert: 0,33 in $CH_2Cl_2/CH_3OH$ 10:1
$\alpha_D$ = 7,4° ( c = 1,04 in Dioxan)

Beispiel 7

N-Octadecyl-N-dodecanoyl-(6-0-methylsulphonyl-ß-D-glucopyranosyl)-amin

9,2 g (0,015 Mol) N-Glucopyranosyl-N-oktadecyldodecansäureamid werden mit 7 g (0,064 mol) Triethylamin in 100 ml Tetrahydrofuran gelöst und bei -20°C Innentemperatur, 3,4 g (0,03 Mol) Methansulfonylchlorid, gelöst in 10 ml Tetrahydrofuran, zugetropft. Man rührt dann 2d bei 20°C, filtriert vom Feststoff ab, und engt das Filtrat im Vakuum zum Sirup ein. Dieses Rohprodukt wird dann säulenchromatographisch auf Kieselgel 60 mit dem Elutionsmittel Toluol-Isopropanol 10:1 gereinigt.
Ausbeute: 4,5 g (43 % der Theorie).
Rf.-Wert = 0,345 (Tol: IPA = 6:1)

Analog können hergestellt werden

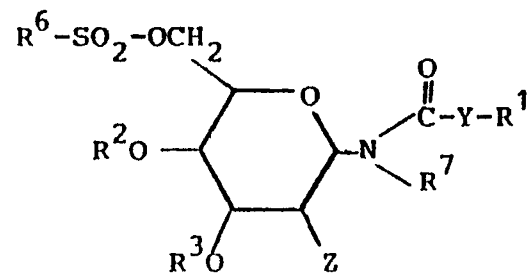

$R^2$, $R^3$ = H

| Beispiel | Zucker | $R^1$ | Y | Z | $R^7$ | $R^6$ | Rf-Wert (Tol: IPA = 6:1)* |
|---|---|---|---|---|---|---|---|
| (8) | Glucose | $(CH_2)_{13}CH_3$ | O | OH | $(CH_2)_{11}CH_3$ | $CH_3$ | 0,310 |
| (9) | Mannose | $(CH_2)_{15}CH_3$ | $CH_2$ | " | $(CH_2)_{13}CH_3$ | $CH_3$ | 0,402 |
| (10) | Glucose | $(CH_2)_9CH_3$ | $CH_2$ | " | $(CH_2)_{17}CH_3$ | 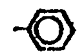 $-CH_3$ | 0,491 |
| (11) | Galactose | $(CH_2)_{15}CH_3$ | $CH_2$ | " | $(CH_2)_{11}CH_3$ | 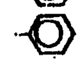 $-CH_3$ | 0,455 |
| (12) | Glucose | $(CH_2)_{15}CH_3$ | $CH_2$ | $NHCOCH_3$ | $(CH_2)_{11}CH_3$ | $CH_3$ | 0,200 |
| (13) | Glucose | $(CH_2)_9CH_3$ | $CH_2$ | " | $(CH_2)_{11}CH_3$ | $CH_3$ | 0,173 |
| (14) | Galactose | $(CH_2)_{15}CH_3$ | $CH_2$ | OH | $(CH_2)_{11}CH_3$ | $CH_3$ | 0,345 |
| (15) | Mannose | $(CH_2)_9CH_3$ | $CH_2$ | OH | $(CH_2)_{17}CH_3$ | $CH_3$ | 0,257 |
| (16) | Mannose | $(CH_2)_9CH_3$ | $CH_2$ | OH | $(CH_2)_{13}CH_3$ | 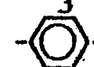 $-CH_3$ | |
| (17) | Glucose | $(CH_2)_{15}CH_3$ | $CH_2$ | OH | $(CH_2)_{13}CH_3$ | 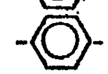 $-CH_3$ | |

*) Thin layer chromatography aluminum foils, Merck, silica gel 60 F 254, 0.2 mm

## Patentansprüche

1. Verbindungen der allgemeinen Formel I

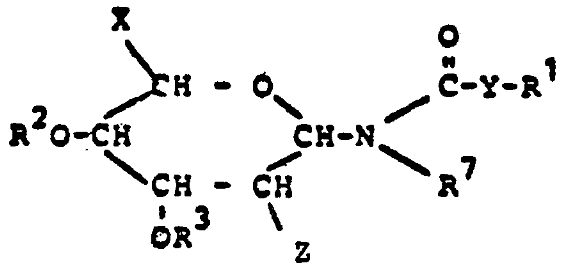

(I)

in der
X für Wasserstoff oder den Rest $-CH_2OR^5$ steht,

Z für $OR^4$ oder -NHV steht, wobei V für Wasserstoff oder $-\overset{O}{\overset{\|}{C}}-W-R^8$ steht,
$R^2$, $R^3$, $R^4$ und $R^5$ gleich oder verschieden sind und für Wasserstoff oder den Rest

$$-\underset{\underset{O}{\|}}{\overset{\overset{O}{\|}}{S}}-R^6$$

stehen, Y und W gleich oder verschieden sind und für Sauerstoff, Schwefel, NH oder $CH_2$ stehen, und $R^1$, $R^6$ $R^7$ und $R^8$ gleich oder verschieden sind und für einen gegebenenfalls substituierten Kohlenwasserstoffrest mit bis zu 50 C-Atomen stehen,
mit der Maßgabe, daß mindestens einer der Reste $R^2$, $R^3$, $R^4$ und $R^5$ die Bedeutung

$$-\underset{\underset{O}{\|}}{\overset{\overset{O}{\|}}{S}}-R^6$$

hat.

2. Verbindungen nach Anspruch 1, in denen die Kohlenwasserstoffreste $R^1$, $R^6$, $R^7$ und $R^8$ bis zu 21 C-Atome aufweisen,

3. Verbindungen nach Anspruch 1, in denen die Kohlenwasserstoffreste $R^1$, $R^7$ 9-21 C-Atome aufweisen, $R^6$ bis zu 7 C-Atome aufweist, und $R^8$ bis zu 21 C-Atome aufweist.

4. Verbindungen nach den Ansprüchen 1-3, in denen $R^2$, $R^3$ und $R^4$ Wasserstoff darstellen und X die Bedeutung $CH_2OR^5$ mit $R^5 = SO_2\ R^6$ und Z die Bedeutung $OR^4$ hat.

5. Verbindung nach den Ansprüchen 1-3, in denen $R^2$ und $R^3$ Wasserstoff darstellen, X die Bedeutung $CH_2OR^5$ mit $R^5 = SO_2R^6$ und Z die Bedeutung $NH-W-R^8$, mit der in den Ansprüchen 1-3 genannten Bedeutung für W und $R^8$ hat.

6. Verfahren zur Herstellung der Verbindungen der allgemeinen Formel I

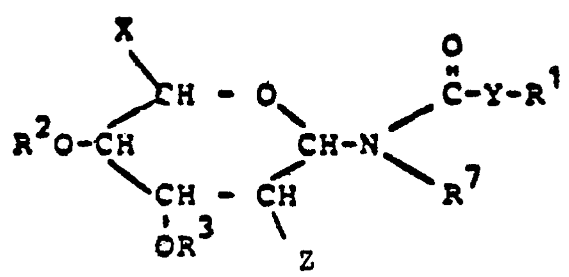

(I)

in der
X für Wasserstoff oder den Rest $-CH_2OR^5$ steht,

Z für $OR^4$ oder -NHV steht, wobei V für Wasserstoff oder $-\overset{O}{\overset{\|}{C}}-W-R^8$ steht,

$R^2$, $R^3$, $R^4$ und $R^5$ gleich oder verschieden sind und für Wasserstoff oder den Rest

$$\begin{array}{c} O \\ \| \\ -\ S\ -\ R^6 \\ \| \\ O \end{array}$$

stehen, und
Y und W gleich oder verschieden sind und für Sauerstoff, Schwefel, NH oder $CH_2$ stehen,
$R^1$, $R^6$, $R^7$, und $R^8$ gleich oder verschieden sind und für einen gegebenenfalls substituierten Kohlenwasserstoffrest mit bis zu 50 C-Atomen stehen,
mit der Maßgabe, daß mindestens einer der Reste $R^2$, $R^3$, $R^4$ und $R^5$ die Bedeutung

$$\begin{array}{c} O \\ \| \\ -\ S\ -\ R^6 \\ \| \\ O \end{array}$$

hat, dadurch gekennzeichnet, daß man eine Verbindung der Formel II

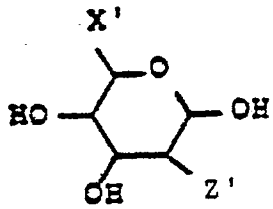

(II)

in der
X' für Wasserstoff oder $CH_2OH$ steht
Z' für NH-CO-W-$R^8$ oder OH steht
entweder in freier, d.h. ungeschützter Form, oder in Form geschützter, gegebenenfalls aktivierter Derivate zunächst mit einer Aminoverbindung $R^7$-$NH_2$, entweder in freier Form oder in Form eines geeigneten Säureadditionssalzes mit der in Anspruch 1 genannten Bedeutung für $R^7$, umsetzt und anschließend das dabei erhaltene Glykosylamin mit einem bei Acylierungsreaktionen üblichen, aktivierten, an funktionellen Gruppen gegebenenfalls geschützten Carbonsäure-, Kohlensäure-, Thiokohlensäurederivat oder Isocyanat am Stickstoffatom acyliert, anschließend in dem so erhaltenen Reaktionsprodukt gegebenenfalls vorhandene Schutzgruppen selektiv oder vollständig abspaltet und in einem zweiten Verfahrensschritt das so erhaltene, an mindestens einer Hydroxygruppe freie, d.h. ungeschützte, Zwischenprodukt mit einem bei Sulfonylierungsreaktionen üblichen, aktivierten, an funktionellen Gruppen gegebenenfalls geschützten Sulfonsäurederivat umsetzt und gegebenenfalls vorhandene Schutzgruppen abspaltet.

7. Verfahren nach Anspruch 6, dadurch gekennzeichnet, daß man in einem ersten Verfahrensschritt einen unblockierten Zucker der Formel II in einem geeigneten Lösungsmittel, gegebenenfalls in Gegenwart eines Katalysators bei Temperaturen zwischen 0°C und 80°C mit 1 bis 10 Äquivalenten des Amins $R^7$-$NH_2$ zum Glykosylamin umsetzt, in einem zweiten Verfahrensschritt dann das Glykosylamin mit 1 bis 10 Äquivalenten eines Acylderivates der Formel $R_1$-Y-CO-X", in der $R_1$ und Y die in Anspruch 1 genannte Bedeutung besitzen und X" Halogen oder eine bei Acylierungsreaktionen gebräuchliche Abgangsgruppe, vorzugsweise einen aktivierenden Esterrest, oder eine Gruppe O-OC-Y-$R_1$ mit der obigen Bedeutung für Y und $R_1$bezeichnet, bzw. 1 bis 10 Äquivalenten eines Isocyanates der Formel $R^1$-NCO umsetzt, wobei man in einem organischen oder wäßrig-organischen Lösungsmittel bei Temperaturen zwischen -30°C und 80°C, gegebenenfalls in Gegenwart einer Base, arbeitet und in einem dritten Verfahrensschritt dann das so erhaltene, am Stickstoff umgesetzte Derivat, in geschützter oder ungeschützeter Form mit 1 bis 10 Äquivalenten eines Sulfonylderivates der Formel

$$\begin{array}{c} O \\ \| \\ R^6\ -\ S\ -\ X''' \\ \| \\ O \end{array} ,$$

in der $R^6$ die in Anspruch 1 genannte Bedeutung besitzt und X''' Halogen bezeichnet, umsetzt, wobei man in einem organischen oder wäßrig-organischen Lösungsmittel bei Temperaturen zwischen -30°C und 80°C, gegebenenfalls in Gegenwart einer Base, arbeitet.

8. Arzneimittel enthaltend mindestens eine Verbindung gemäß Anspruch 1.

9. Verwendung der Verbindungen gemäß Anspruch 1 zur Herstellung von Arzneimitteln.

10. Verbindungen der allgemeinen Formel I

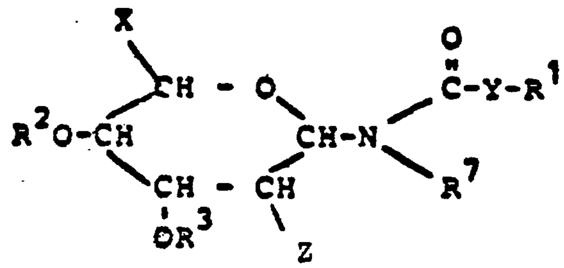

(I)

in der

X für Wasserstoff oder den Rest $-CH_2OR^5$ steht,

Z für $OR^4$ oder -NHV steht, wobei V für Wasserstoff oder 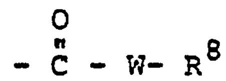 steht,

$R^2$, $R^3$, $R^4$ und $R^5$ gleich oder verschieden sind und für Wasserstoff oder den Rest

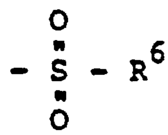

stehen, und

Y und W gleich oder verschieden sind und für Sauerstoff, Schwefel, NH oder $CH_2$ stehen,

$R^1$, $R^6$, $R^7$, und $R^8$ gleich oder verschieden sind und für einen gegebenenfalls substituierten Kohlenwasserstoffrest mit bis zu 50 C-Atomen stehen,

mit der Maßgabe, daß mindestens einer der Reste $R^2$, $R^3$, $R^4$ und $R^5$ die Bedeutung

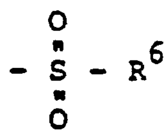

hat, zur Anwendung in einem Verfahren zur therapeutischen Behandlung des menschlichen oder tierischen Körpers.

## Claims

1. Compunds of the general formula I

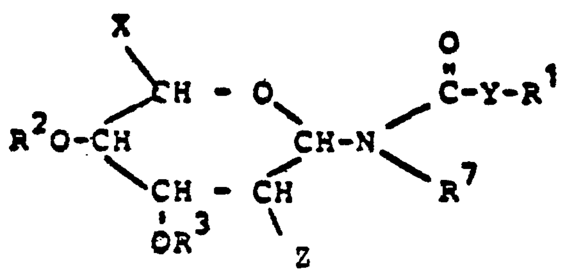

(I)

in which

X represents hydrogen or the radical $-CH_2OR^5$,

Z represents $OR^4$ or -NHV,

wherein

V represents hydrogen or 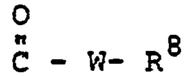

$R^2$, $R^3$, $R^4$ and $R^5$ are identical or different and represent hydrogen or the radical

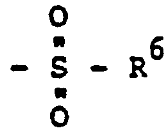

Y and W are identical or different and represent oxygen, sulphur, NH or $CH_2$, and

$R^1$, $R^6$, $R^7$ and $R^8$ are identical or different and represent an optionally substituted hydrocarbon radical with up to 50 C atoms, with the proviso that at least one of the radicals $R^2$, $R^3$, $R^4$ and $R^5$ denotes

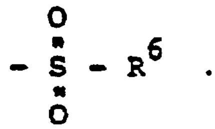

2. Compounds according to Claim 1, in which the hydrocarbon radicals $R^1$, $R^6$, $R^7$ and $R^8$ have up to 21 C atoms.

3. Compunds according to Claim 1, in which the hydrocarbon radicale $R^1$ and $R^7$ have 9–21 C atoms, $R^6$ has up to 7 C atoms and $R^8$ has up to 21 C atoms.

4. Compunds according to Claims 1–3, in which $R^2$, $R^3$ and $R^4$ represent hydrogen and X denotes $CH_2OR^5$, where $R^5 = SO_2R^6$, and Z denotes $OR^4$.

5. Compunds according to Claims 1–3, in which $R^2$ and $R^3$ represent hydrogen, X denotes $CH_2OR^5$, where $R^5 = SO_2R^6$, and Z denotes NH–W–$R^8$, with the meaning of W and $R^8$ given in Claims 1–3.

6. Process for the preparation of the compounds of the general formula I

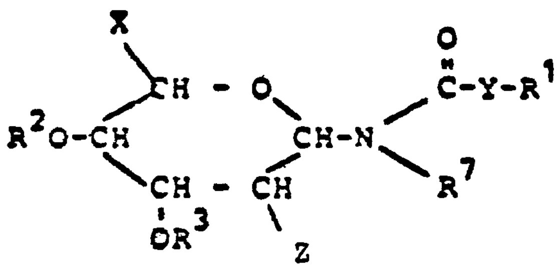

(I)

in which

X represents hydrogen or the radical $-CH_2OR^5$,

Z represents $OR^4$ or -NHV,

wherein

V represents hydrogen or 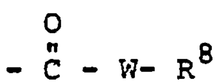

$R^2$, $R^3$, $R^4$ and $R^5$ are identical or different and represent hydrogen or the radical

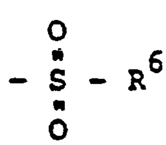

and Y and W are identical or different and represent oxygen, sulphur, NH or $CH_2$,

$R^1$, $R^6$, $R^7$ and $R^8$ are identical or different and represent an optionally substituted hydrocarbon radical with up to 50 C atoms, with the proviso that at least one of the radicals $R^2$, $R^3$, $R^4$ and $R^5$ denotes

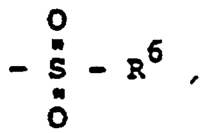

characterized in that a compund of the formula II

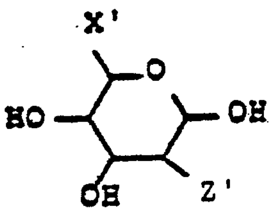

(II)

in which
X' represents hydrogen or $CH_2OH$ and
Z' represents $NH-CO-W-R^8$ or OH,
is first reacted, either in the free, that is to say unprotected, form or in the form of protected, optionally activated derivatives, with an amino compound $R^7-NH_2$, either in the free form or in the form of a suitable acid addition salt, with the meaning of $R^7$ given in Claim 1, and the glycosylamine thereby obtained is then acylated on the nitrogen atom with an activated carboxylic acid, carbonic acid or thiocarbonic acid derivative or isocyanate which is customary in acylation reactions and, if appropriate, is protected on functional groups, any protective groups present in the reaction product thus obtained are then split off selectively or completely, and, in a second process step, the intermediate product thus obtained, which is in the free state, that is to say unprotected, on at least one hydroxyl group, is reacted with an activated sulphonic acid derivative which is customary in sulphonylation reactions and, if appropriate, is protected on functional groups, and any protective groups present are split off.

7. Process according to Claim 6, characterized in that, in a first process step, an unblocked sugar of the formula II is reacted with 1 to 10 equivalents of the amine $R^7-NH_2$ in a suitable solvent, if appropriate in the presence of a catalyst, at temperatures between 0°C and 80°C to give the glycosylamine, the glycosylamine is then reacted, in a second process step, with 1 to 10 equivalents of an acyl derivative of the formula $R_1-Y-CO-X''$, in which $R_1$ and Y have the meaning given in claim 1 and X" designates halogen or a leaving group customary in acylation reactions, preferably an activating ester radical, or a group $O-OC-Y-R_1$, with the above meaning of Y and $R_1$, or 1 to 10 equivalents of an isocyanate of the formula $R^1-NCO$, the reaction being carried out in an organic or aqueous-organic solvent at temperatures between −30°C and 80°C, if appropriate in the presence of a base, and, in a third process step, the derivative thus obtained, which has been reacted on the nitrogen, is reacted, in the protected or unprotected form, with 1 to 10 equivalents of a sulphonyl derivative of the formula

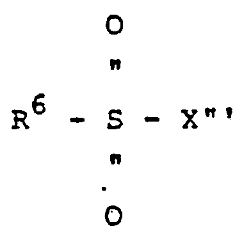

in which
$R^6$ has the meaning given in Claim 1 and
X''' designates halogen,
the reaction being carried out in an organic or aqueous-organic solvent at temperatures between −30°C and 80°C, if appropriate in the presence of a base.

8. Medicament containing at least one compund according to Claim 1.

9. Use of the compounds according to Claim 1 for the preparation of medicaments.

10. Compounds of the general formula I

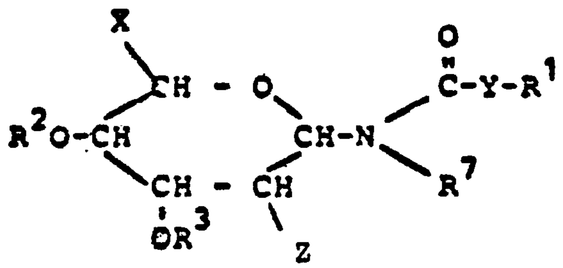

(I)

in which
X represents hydrogen or the radical $-CH_2OR^5$,
Z represents $OR^4$ or $-NHV$,
wherein

V represents hydrogen or $-\overset{O}{\overset{\|}{C}}-W-R^8$
$R^2$, $R^3$, $R^4$ and $R^5$ are identical or different and represent hydrogen or the radical

$$-\overset{O}{\overset{\|}{\underset{\underset{O}{\|}}{S}}}-R^6$$

and
Y and W are identical or different and represent oxygen, sulphur, NH or $CH_2$,
$R^1$, $R^6$, $R^7$ and $R^8$ are identical or different and represent an optionally substituted hydrocarbon radical with up to 50 C atoms,
with the proviso that at least one of the radicals $R^2$, $R^3$, $R^4$ and $R^5$ denotes

$$-\overset{O}{\overset{\|}{\underset{\underset{O}{\|}}{S}}}-R^6$$

for use in a process for the therapeutic treatment of the human or animal body.

**Revendications**

1. Composés de formule générale I

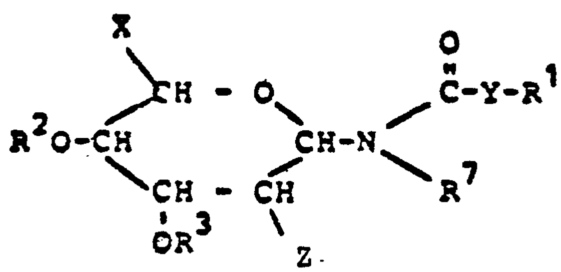

(I)

dans laquelle
X représente l'hydroxygène ou un reste $-CH_2OR^5$.
Z représente un groupe $OR^4$ ou $-NHV$,
V preprésentant l'hydrogène ou $-\overset{O}{\overset{\|}{C}}-W-R^8$
$R^2$. $R^3$, $R^4$ et $R^5$ sont identiques ou différents et représentent l'hydrogène ou un reste

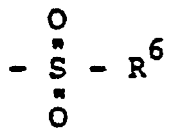

Y et W sont identiques ou différents et représentent l'oxygène, le soufre, NH ou $CH_2$, et
$R^1$, $R^6$, $R^7$et $R^8$ sont identiques ou différents et représentent un reste d'hydrocarbure éventuellement subsitué avec jusqu'à 50 atomes de carbone, avec la condition qu'au moins un des restes $R^2$, $R^3$, $R^4$ et $R^5$ ait la signification

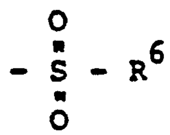

2. Composés selon la revendication 1, dans lesquels les restes d'hydrocarbures $R^1$, $R^6$, $R^7$ et $R^8$ possèdent jusqu'à 21 atomes de carbone.
3. Composés selon la revendication 1, dans lesquels les restes d'hydrocarbures $R^1$, $R^7$ possèdent 9–21 atomes de carbone, $R^6$ possède jusqu'à 7 atomes de carbone, et $R^8$ possède jusqu'à 21 atomes de carbone.
4. Composés selon les revendications 1–3, dans lesquels $R^2$, $R^3$ et $R^4$ représentent l'hydrogène et X a la signification $CH_2OR^5$ avec $R^5 = SO_2R^6$ et Z a la signification $OR^4$.
5. Composés selon les revendications 1–3, dans lesquels $R^2$ et $R^3$ représentent l'hydrogène, X a la signification $CH_2OR^5$ avec $R^5 = SO_2R^6$ et Z a la signification NH–W–$R^8$, W et $R^8$ ayant les significations décrites dans les revendications 1–3.
6. Procédé pour la fabrication des composés de formule générale I

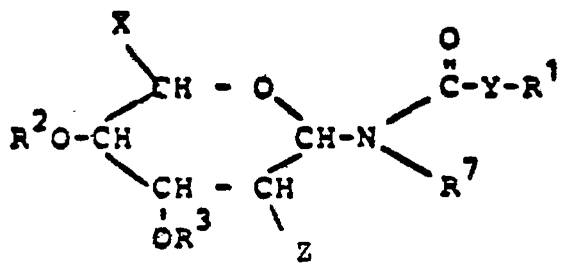
(I)

dans laquelle
X représente l'hydrogène ou un reste -$CH_2OR^5$,
Z représente $OR^4$ ou -NHV, V représentant l'hydrogène ou 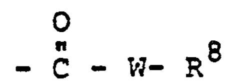
$R^2$, $R^3$, $R^4$ et $R^5$ sont identiques ou différents et représentent l'hydrogène ou un reste

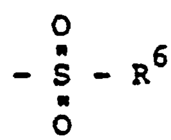

et
Y et W sont identiques ou différents et représentent l'oxygène, le soufre, NH ou $CH_2$, et
$R^1$, $R^6$, $R^7$ et $R^8$ sont identiques ou différents et représentent un reste d'hydrocarbure éventuellement substitué avec jusqu'à 50 atomes de carbone,
avec la condition qu'au moins un des restes $R^2$, $R^3$, $R^4$ et $R^5$ ait la signification

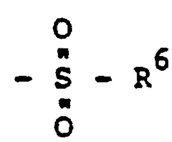

caractérisé en ce que l'on fait d'abord réagir un composé de formule II

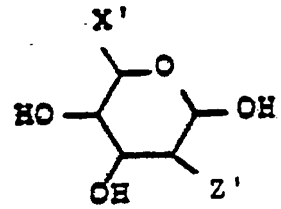

(II)

dans laquelle
X' représente l'hydrogène ou $CH_2OH$,
Z' représente $NH-CO-W-R^8$ ou OH
soit sous forme libre, c'est-à-dire non protégée, soit sous forme de dérivés protégés, éventuellement activés, avec un composé amino $R^7-NH_2$, soit sous forme libre, soit sous forme d'un sel d'addition d'acide approprié, avec pour $R^7$ la signification décrite dans la revendication 1, puis qu'on acyle à l'atome d'azote la glycosylamine ainsi obtenue avec un isocyanate ou un dérivé d'acide carboxylique, d'acide carbonique, thiocarbonique usuel des réactions d'acylation, activé, éventuellement protégé sur des groupes fonctionnels, puis qu'on sépare sélectivement ou totalement les groupes de protection éventuellement présents dans le produit réactionnel ainsi obtenu et que, dans une seconde étape du procédé, on fait réagir le produit intermédiaire ainsi obtenu, libre, c'est-à-dire non protégé, en au moins un groupe hydroxy, avec un dérivé d'acide sulfonique usuel des réactions de sulfonylation, activé, éventuellement protégé aux groupements fonctionnels, et qu'on sépare les groupes de protection éventuellement présents.

7. Procédé selon la revendication 6, caractérisé en ce qu'on fait réagir, dans une première étape de procédé, un sucre non bloqué de formule II dans un solvant approprié, éventuellement en présence d'un catalyseur, à des températures comprises dans l'intervalle de 0°C à 80°C, avec 1 à 10 équivalents de l'amine $R^7-NH_2$ de manière à obtenir la glycosylamine, puis qu'on fait réagir dans une deuxième étape de procédé la glycosylamine avec 1 à 10 équivalents d'un dérivé acylé de formule $R_1-Y-CO-X''$, dans laquelle $R_1$ et Y ont les significations décrites dans la revendication 1 et X'' représente un halogène ou un groupe séparable usuel des réactions d'acylation, de préférence un reste d'ester activant, ou un groupe $O-OC-Y-R_1$ dans lequel Y et $R_1$ ont les significations ci-dessus, ou 1 à 10 équivalents d'un isocyanate de formule $R^1-NCO$, en travaillant dans un solvant organique ou aqueux-organique à des températures dans l'intervalle de −30°C à +80°C, éventuellement en présence d'une base, et que, dans une troisième étape, on fait réagir le dérivé modifié sur l'azote ainsi obtenu, sous forme protégé ou non protégée avec 1 à 10 équivalents d'un dérivé sulfonylé de formule

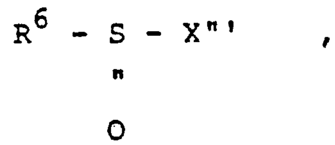

dans laquelle $R^6$ possède les significations décrites dans la revendication 1 et X''' représente un halogène, en travaillant dans un solvant organique ou aqueux-organique à des températures dans l'intervalle de −30°C à 80°C, éventuellement en présence d'une base.

8. Médicament contenant au moins un composé selon la revendication 1.

9. Utilisation des composés selon la revendication 1 pour la fabrication de médicaments.

10. Composés de formule générale I

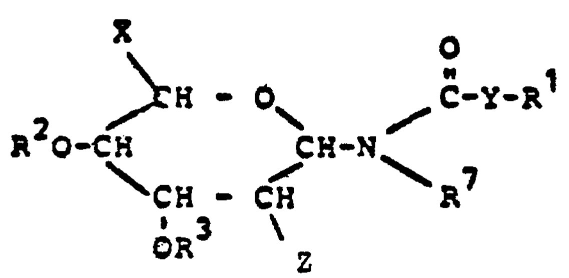

(I)

dans laquelle
X représente l'hydrogène ou un reste $-CH_2-OR^5$,
Z représente $OR^4$ ou -NHV, V représentant l'hydrogène ou 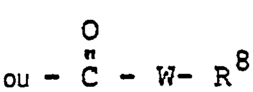

$R^2$, $R^3$, $R^4$ et $R^5$ sont identiques ou différents et représentent l'hydrogène ou un reste

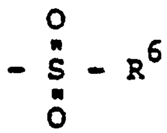

et

Y et W sont identiques ou différents et représentent l'oxygène, le soufre, NH ou $CH_2$, et

$R^1$, $R^6$, $R^7$ et $R^8$ sont identiques ou différents et représentent un reste d'hydrocarbure éventuellement substitué avec jusqu'à 50 atomes de carbone, avec la condition qu'au moins un des restes $R^2$, $R^3$, $R^4$ et $R^5$ ait la signification

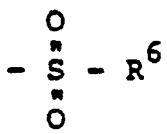

pour utilisation dans un procédé destiné au traitement thérapeutique du corps humain ou animal.